# FASCICULE DE BREVET EUROPEEN

(11) **EP 4 472 778 B1**
(45) Date de publication et mention de la délivrance du brevet: **04.02.2026**
(21) Numéro de dépôt: 22844251.3
(22) Date de dépôt: 09.12.2022
(51) Int. Cl.: B05B 11/10, A61M 11/00, A61M 15/08, B05B 11/00

(54) **DISPOSITIF DE DISTRIBUTION DE PRODUIT FLUIDE ET SON PROCEDE D'AMORÇAGE**
VORRICHTUNG ZUR ABGABE EINES FLÜSSIGEN PRODUKTS UND VERFAHREN ZUM FÜLLEN DAVON
DEVICE FOR DISPENSING A FLUID PRODUCT AND METHOD FOR PRIMING SAME

(30) Priorité: 31.01.2022 FR 2200837
(43) Date de publication de la demande: 11.12.2024
(73) Titulaire: APTAR France SAS, 27110 Le Neubourg (FR)
(72) Inventeur: PETIT, Ludovic, 27110 VITOT (FR); SAVALLE, Matthieu, 76350 OISSEL (FR)
(74) Mandataire: CAPRI
(86) Numéro de dépôt international: PCT/FR2022/052304
(87) Numéro de publication internationale: WO 2023/144468

(56) Documents cités:
- DE-U1- 20 218 974
- US-A1- 2008 149 098
- US-A1- 2010 032 499

## Description

La présente invention concerne un dispositif de distribution de produit fluide, notamment pour une distribution nasale d'une ou deux doses de produit fluide pharmaceutique. La présente invention concerne également un procédé d'amorçage d'un dispositif de distribution de produit fluide.

Un inconvénient de certains dispositifs de distribution nasale de produit fluide, en particulier ceux utilisant une pompe avec une chambre de dosage pour définir le volume de la dose distribuée à chaque actionnement, concerne l'obligation d'amorcer la pompe pour remplacer l'air contenu initialement dans la chambre de dosage par du fluide. Cet amorçage est généralement réalisé par un ou plusieurs actionnements du dispositif, avec par conséquent une perte possible de produit fluide. US 2010/032499 divulgue un dispositif de distribution nasale de produit fluide.

La présente invention a pour but de fournir un dispositif de distribution de produit fluide et son procédé d'amorçage qui ne reproduisent pas les inconvénients susmentionnés.

Plus particulièrement, la présente invention a pour but de fournir un tel dispositif et procédé qui ne nécessitent aucun amorçage de la pompe.

La présente invention a également pour but de fournir un tel dispositif et procédé qui soient simples et peu coûteux à fabriquer et à assembler.

La présente invention a donc pour objet un dispositif de distribution de produit fluide comprenant un distributeur assemblé sur un réservoir contenant du produit fluide, ledit réservoir contenant plusieurs doses de produit fluide distribuées en plusieurs actionnements successifs, ledit distributeur comportant un corps de pompe contenant une chambre de dosage, et une tête de distribution comportant un orifice de distribution, ledit orifice de distribution comportant un obturateur déplaçable et/ou déformable entre une position de fermeture et une position d'ouverture, ledit obturateur étant élastiquement sollicité vers sa position de fermeture et étant adapté à s'ouvrir lors de l'actionnement, ladite tête de distribution étant déplaçable axialement par rapport audit corps de pompe entre une position de repos et une position d'actionnement, un piston, solidaire de ladite tête de distribution, étant adapté à coulisser dans ladite chambre de dosage entre une position de repos et une position d'actionnement, caractérisé en ce que ladite chambre de dosage comporte trois ouvertures, une première ouverture reliant ladite chambre de dosage audit réservoir lorsque ledit piston est en position de repos avant le premier actionnement, une seconde ouverture reliant ladite chambre de dosage audit orifice de distribution et une troisième ouverture prévue à l'extrémité axiale inférieure dudit corps de pompe, lesdites première et troisième ouvertures étant isolées de ladite chambre de dosage lorsque ledit piston se déplace hors de sa position de repos, ladite chambre de dosage étant remplie à travers ladite première ouverture lorsque ledit distributeur est assemblé sur ledit réservoir.

Avantageusement, ladite première ouverture est réalisée dans une partie cylindrique dudit corps de pompe dans laquelle ledit piston coulisse lors de l'actionnement.

Avantageusement, un bord inférieur de ladite première ouverture est disposé au repos à une distance d1 dudit piston, de sorte que, lors de l'actionnement, ladite première ouverture est fermée après une course initiale correspondant à ladite distance d1.

Avantageusement, ladite distance d1 forme moins de 30% de la course d'actionnement totale dudit piston, de préférence environ 20%.

Avantageusement, ladite première ouverture comporte une ou plusieurs fentes formée(s) dans la partie cylindrique dudit corps de pompe.

Avantageusement, une tige creuse est disposée dans ladite tête de distribution, ladite tige creuse définissant un canal d'expulsion reliant lors de l'actionnement ladite chambre de dosage audit orifice de distribution, ladite tige creuse comportant ledit piston.

Avantageusement, ledit piston est formé par un joint, notamment un joint torique, assemblé sur ladite tige creuse.

Avantageusement, ladite troisième ouverture comporte un organe d'obturation, tel qu'une bille, formant un clapet d'entrée pour ladite chambre de dosage permettant le remplissage de celle-ci après chaque actionnement.

Avantageusement, le dispositif comporte une membrane filtrante pour filtrer l'air d'éventation après chaque actionnement.

Avantageusement, le dispositif comporte des moyens anti-actionnement pour empêcher le déplacement axial de ladite tête de distribution par rapport audit corps de pompe.

Selon une première variante avantageuse, lesdits moyens anti-actionnement comportent un clip amovible disposé autour dudit distributeur avant le premier actionnement.

Selon une seconde variante avantageuse, lesdits moyens anti-actionnement comportent une projection radiale solidaire dudit corps de pompe coopérant avec une rainure horizontale d'un profil de rainures solidaire de ladite tête de distribution.

Avantageusement, le dispositif comporte des moyens de limitation de course d'actionnement pour réduire la course d'actionnement dudit piston après la distribution de la première dose en décalant axialement la position de repos dudit piston après le premier actionnement.

Selon une première variante avantageuse, lesdits moyens de limitation de course d'actionnement comportent une lèvre radiale solidaire dudit corps de pompe coopérant avec un épaulement radial solidaire dudit piston.

Selon une seconde variante avantageuse, lesdits moyens de limitation de course d'actionnement comportent une projection radiale solidaire dudit corps de pompe coopérant avec un profil de rainures solidaire de ladite tête de distribution.

Avantageusement, ledit profil de rainures comporte une première rainure verticale et une seconde rainure verticale parallèle, reliées par une rainure oblique, la position de repos dudit piston avant le premier actionnement correspondant à ladite projection radiale disposée dans l'extrémité axiale inférieure de ladite première rainure verticale et la position de repos dudit piston après le premier actionnement correspondant à ladite projection radiale disposée dans l'extrémité axiale inférieure de ladite seconde rainure verticale, lesdites extrémités axiales inférieures étant décalées axialement l'une de l'autre.

Avantageusement, ladite première rainure verticale comporte une partie de paroi déformable adaptée à guider ladite projection radiale vers ladite rainure oblique lorsque ledit piston revient vers sa position de repos après distribution de la première dose.

Avantageusement, ladite rainure oblique comporte une seconde partie de paroi déformable adaptée à empêcher ladite projection radiale de revenir dans ladite rainure oblique lorsque ladite projection radiale est entrée dans ladite seconde rainure verticale.

Avantageusement, ledit réservoir contient deux doses de produit fluide distribuées en deux actionnements successifs.

La présente invention a également pour objet un procédé d'amorçage d'un dispositif de distribution de produit fluide, les étapes suivantes:
- fournir un réservoir contenant au moins deux doses de produit fluide,
- fournir un distributeur comportant un corps de pompe contenant une chambre de dosage, et une tête de distribution comportant un orifice de distribution, ledit orifice de distribution comportant un obturateur déplaçable et/ou déformable entre une position de fermeture et une position d'ouverture, ledit obturateur étant élastiquement sollicité vers sa position de fermeture et étant adapté à s'ouvrir lors de l'actionnement, ladite tête de distribution étant déplaçable axialement par rapport audit corps de pompe entre une position de repos et une position d'actionnement, un piston, solidaire de ladite tête de distribution, étant adapté à coulisser dans ladite chambre de dosage entre une position de repos et une position d'actionnement, ladite chambre de dosage comportant trois ouvertures, une première ouverture reliant ladite chambre de dosage audit réservoir lorsque ledit piston est en position de repos avant le premier actionnement, une seconde ouverture reliant ladite chambre de dosage audit orifice de distribution et une troisième ouverture prévue à l'extrémité axiale inférieure dudit corps de pompe, lesdites première et troisième ouvertures étant isolées de ladite chambre de dosage lorsque ledit piston se déplace hors de sa position de repos,
ledit procédé comportant l'étape d'assembler ledit distributeur sur ledit réservoir, en insérant ledit corps de pompe dans ledit réservoir avec ledit piston en position de repos, cette insertion déplaçant le produit fluide contenu dans ledit réservoir, ledit produit fluide déplacé pénétrant dans ladite chambre de dosage à travers ladite première ouverture, pour ainsi amorcer le dispositif.

Ces caractéristiques et avantages et d'autres de la présente invention apparaitront plus clairement au cours de la description détaillée suivante, faite en référence aux dessins joints, donnés à titre d'exemples non limitatifs, et sur lesquels :
La figure 1 est une vue schématique en section transversale d'un dispositif de distribution de produit fluide selon un premier exemple non couvert par les revendications, lors de l'assemblage du distributeur sur le réservoir,
La figure 2 est une vue similaire à celle de la figure 1, lors du premier actionnement,
La figure 3 est une vue similaire à celle de la figure 2, après le premier actionnement,
La figure 4 est une vue similaire à celle de la figure 3, après chargement de la chambre de dosage avec une nouvelle dose de produit fluide,
La figure 5 est une vue schématique agrandie d'une partie de la figure 1,
La figure 6 est une vue schématique découpée d'un réservoir avant assemblage du distributeur,
La figure 7 est une vue schématique en perspective d'un ressort plastique pouvant remplacer le ressort métallique des figures 1 à 4,
La figure 8 est une vue schématique en section transversale d'un dispositif de distribution de produit fluide selon un premier mode de réalisation selon les revendications, après assemblage du distributeur sur le réservoir,
La figure 9 est une vue similaire à celle de la figure 8, lors du premier actionnement,
La figure 10 est une vue similaire à celle de la figure 9, après chargement de la chambre de dosage avec une nouvelle dose de produit fluide,
La figure 11 est une vue schématique en section transversale d'un dispositif de distribution de produit fluide selon un deuxième mode de réalisation selon les revendications, après assemblage du distributeur sur le réservoir,
La figure 12 est une vue similaire à celle de la figure 11, lors du premier actionnement,
La figure 13 est une vue similaire à celle de la figure 12, après chargement de la chambre de dosage avec une nouvelle dose de produit fluide,
La figure 14 est une vue schématique agrandie d'une partie de la figure 13,
Les figures 15 à 17 sont des vues similaires à celles des figures 11 à 13, illustrant une variante de réalisation avantageuse du deuxième mode de réalisation,
La figure 18 est une vue schématique en section transversale d'un dispositif de distribution de produit fluide selon un deuxième exemple non couvert par les revendications, montrant une solution alternative pour réaliser les fonctions d'anti-actionnement et de modification de volume de dose après la première dose, en position actionnée,
La figure 19 est une vue schématique de détail du deuxième exemple, et
La figure 20 est vue agrandie du profil fonctionnel de la figure 19.

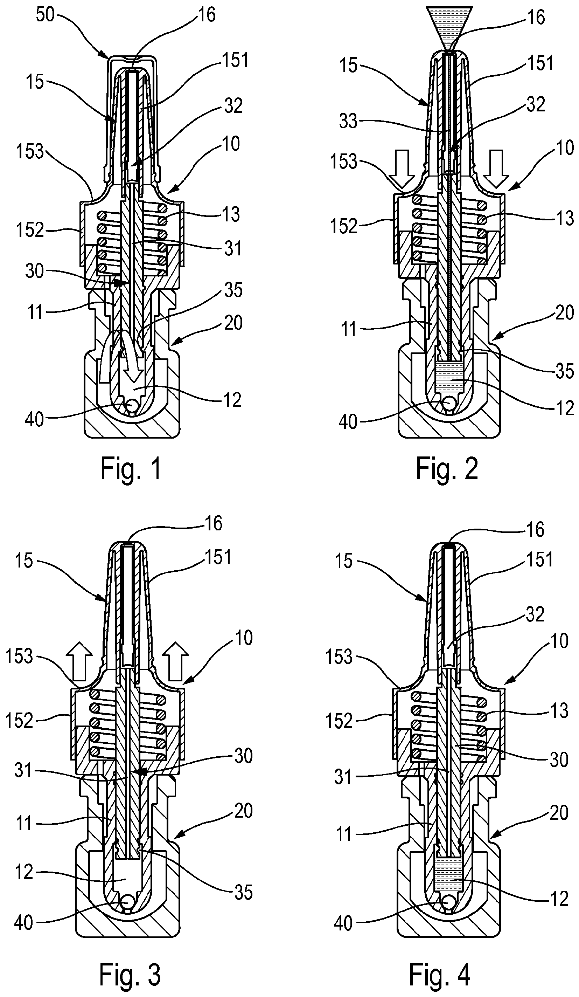

Les termes "axial" et "radial" se réfèrent à l'axe central longitudinal du dispositif. Les termes "amont" et "aval" se réfèrent à la direction d'écoulement du fluide lors de l'actionnement. Les termes "haut" et "bas" se réfèrent à la position droite du dispositif représentée sur les figures 1 à 4, 8 à 13 et 15 à 18.

Dans un premier exemple des figures 1 à 5, un distributeur 10 est assemblé sur un réservoir 20 contenant du produit fluide à distribuer, typiquement un liquide de type pharmaceutique.

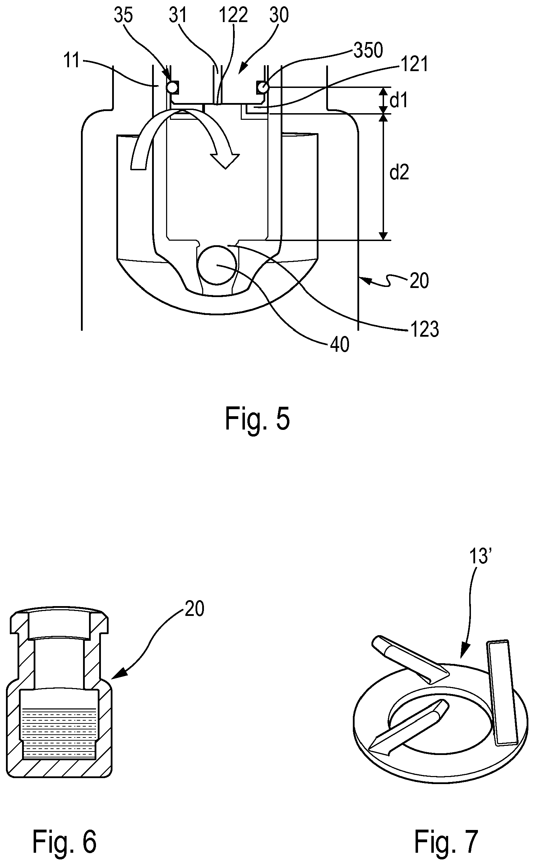

Le réservoir 20 est avantageusement formé par un corps creux et borgne, comportant une seule ouverture et contenant une ou plusieurs dose(s) de produit fluide à distribuer. Un exemple de réalisation de ce réservoir 20 adapté à l'exemple des figures 1 à 5 est représenté de manière isolée sur la figure 6.

La présente invention s'applique à un dispositif unidose ne contenant qu'une seule dose de produit fluide dans le réservoir 20, mais elle est particulièrement adaptée pour des dispositifs dits bidoses, où le réservoir 20 contient deux doses de produit fluide, distribuées en deux actionnements successifs. Eventuellement, le réservoir 20 pourrait contenir plus de deux doses, par exemple trois ou quatre doses.

Le distributeur 10 comprend un corps de pompe 11 contenant une chambre de dosage 12, et une tête de distribution 15 comportant un orifice de distribution 16, de préférence orienté axialement. Cet orifice de distribution 16 sert à distribuer une dose de produit fluide hors de ladite tête de distribution 15 lors de l'actionnement du dispositif par un utilisateur. La tête de distribution 15 est déplaçable axialement par rapport au corps de pompe 11 entre une position de repos et une position d'actionnement.

Au repos, un capuchon de protection amovible 50 peut être disposé sur la tête de distribution 15 pour protéger l'orifice de distribution 16, comme visible sur les figures 1, 8 et 11.

Un ressort 13 est disposé entre le corps de pompe 11 et la tête de distribution 15 pour solliciter cette dernière vers sa position de repos.

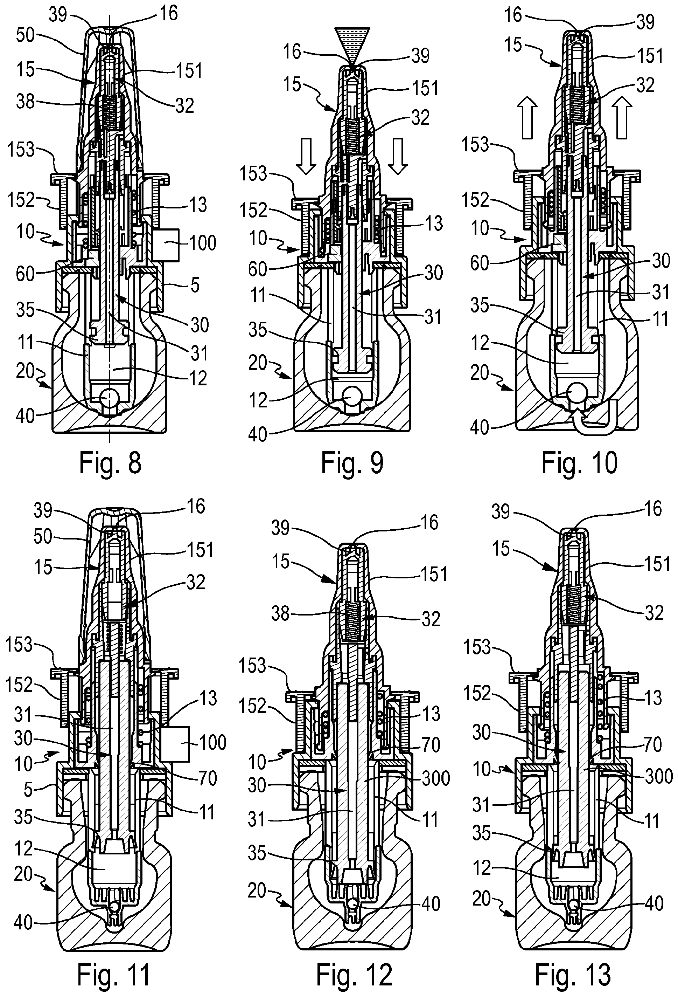

Dans l'exemple des figures 1 à 4 et dans les modes de réalisation des figures 8 à 17, le ressort 13 est du type classique à boudin, généralement métallique.

En variante, on peut envisager de remplacer le ressort 13 par un élément élastiquement déformable 13' en plastique. La figure 7 montre un exemple d'un tel élément élastique 13'.

La tête de distribution 15 comporte un embout nasal allongé 151 comportant à son extrémité axiale ledit orifice de distribution 16, ainsi qu'un corps latéral 152, relié audit embout nasal 151 par une bride 153 environ radiale. En variante, comme visible sur les figures 8 à 17, le corps latéral 152 et la bride 153 peuvent être formés sur une pièce séparée assemblée sur la tête de distribution 15.

Une tige creuse 30 est connectée à la tête de distribution 15, ladite tige creuse 30 définissant un canal d'expulsion 31.

Un insert 32, avantageusement creux, peut être disposé dans la tête de distribution 15 pour définir en amont de l'orifice de distribution 16 une partie de canal d'expulsion 33 de dimension radiale réduite. Ceci permet non seulement de limiter le volume mort, mais aussi de réaliser une bonne pulvérisation à travers l'orifice de distribution 16.

Avantageusement, un profil de pulvérisation peut être prévu directement en amont dudit orifice de distribution 16, entre ledit insert 32 et la paroi de fond de la tête de distribution 15.

Du côté opposé, la tige 30 comporte un piston 35 adapté à coulisser dans la chambre de dosage 12 du corps de pompe 11 entre une position de repos et une position d'actionnement. Par exemple, un joint 350, notamment un joint torique, peut être assemblé sur la tige creuse 30 pour former le piston, comme plus particulièrement visible sur la figure 5. En variante, le piston 35 pourrait être formé monobloc avec la tige creuse 30. D'autres variantes connues sont aussi envisageables pour réaliser le piston 35.

L'assemblage du distributeur 10 sur le réservoir 20 peut être réalisé d'une quelconque manière connue, par exemple en utilisant une bague de fixation pour fixer le corps de pompe 11 sur un col du réservoir.

La chambre de dosage 12, selon les revendications, comporte trois ouvertures 121, 122, 123. Ces ouvertures sont distinctes, et ont chacune une fonction spécifique.

Dans le cas d'un unidose, qui n'est pas selon les revendications, lorsque le réservoir 20 ne contient qu'une seule dose de produit fluide, il y a deux ouvertures 121 et 122.

Dans le cas d'un bidose ou multidose, lorsque le réservoir 20 contient au moins deux doses de produit fluide, il y a trois ouvertures 121, 122 et 123.

La première ouverture 121 est réalisée dans la partie cylindrique du corps de pompe 11 dans laquelle le piston 35 coulisse lors de l'actionnement.

Cette première ouverture 121 relie la chambre de dosage 12 au réservoir 20 lorsque le piston 35 est en position de repos. Lors de l'actionnement, le piston 35 coulisse axialement vers le bas dans le corps de pompe 11. Eventuellement, notamment dans le cas d'un bidose, après distribution de la première dose, le piston 35 remonte axialement pour revenir dans sa position de repos. A la fin de cette course de retour, la première ouverture 121 est ouverte à nouveau.

Comme visible sur la figure 5, la première ouverture 121 est de préférence disposée au repos à proximité du piston 35, de sorte qu'après une petite course d'actionnement initiale d1, illustrée sur la figure 5, le piston 35 dépasse la première ouverture 121, et pour la suite de la course d'actionnement d2, cette première ouverture 121 est donc fermée. En d'autres termes, un bord inférieur de la première ouverture 121 est disposé au repos à une distance d1 du piston 35, de sorte que, lors de l'actionnement, ladite première ouverture 121 est fermée après une course initiale correspondant à ladite distance d1. Avantageusement, la distance d1 forme moins de 30% de la course d'actionnement totale d1 + d2 du piston 35, de préférence environ 20%.

Avantageusement, la première ouverture 121 comporte une ou plusieurs fentes formées dans la partie cylindrique du corps de pompe 11. Le nombre de fentes, qui pourraient aussi être des trous, peut être quelconque, par exemple un, deux, trois ou quatre fentes.

La seconde ouverture 122 est axiale et est prévue au niveau de l'extrémité axiale inférieure de la tige creuse 30. La seconde ouverture 122 relie ainsi la chambre de dosage 12 au canal d'expulsion 31.

La troisième ouverture 123 est axiale et est prévue à l'extrémité axiale inférieure du corps de pompe 11. Cette troisième ouverture n'est prévue que si le dispositif est un bidose ou un multidose. Un organe d'obturation 40, tel qu'une bille, est prévu pour fermer la troisième ouverture 123 dès le début de l'actionnement, et pour ouvrir la troisième ouverture 123 dès le début de la course de retour du piston 35, quand celui-ci revient de sa position d'actionnement vers sa position de repos. Cette troisième ouverture 123 ensemble avec l'organe d'obturation 40 forme donc un clapet d'entrée classique pour la chambre de dosage 12, permettant le remplissage de celle-ci après chaque actionnement de la pompe du fait de la dépression générée par le piston 35 qui remonte vers sa position de repos.

Dans l'exemple représenté sur les figures 1 à 4, la chambre de dosage 12 ne comporte pas de clapet de sortie au niveau de la seconde ouverture 122, et l'orifice de distribution 16 ne comporte pas d'obturateur formant clapet de sortie. La chambre de dosage 12 est donc reliée en permanence à l'atmosphère. Néanmoins, le rapport des aires des sections transversale du canal d'expulsion 31 et de la troisième ouverture 123 est tel, que la dépression est générée quand même dans la chambre de dosage 12 lorsque le piston 35 retourne vers sa position de repos. Ainsi par exemple, le diamètre du canal d'expulsion 31 peut être de 0,3 mm, ce qui donne une aire de la section transversale de 0,7 mm², alors que le diamètre de la troisième ouverture 123 est typiquement d'environ 3 mm, ce qui donne une aire de la section transversale de 7 mm², soit un rapport de 1 à 100. Néanmoins, en variante, on pourrait envisager un clapet de sortie au niveau de la seconde ouverture 122 de la chambre de dosage, ou, dans une variante selon les revendications, un obturateur au niveau de l'orifice de distribution 16, comme dans les modes de réalisation décrits ci-après en référence aux figures 8 à 17.

Ainsi, l'invention permet de réaliser un dispositif sans amorçage.

Lors de l'assemblage du distributeur 10 sur le réservoir 20, la tête de distribution 15 et le piston 35 sont en position de repos par rapport au corps de pompe 11. La première ouverture 121 est donc ouverte. Ainsi, lorsque le distributeur 10 est assemblé sur le réservoir 20, le corps de pompe 11 pénètre dans le réservoir 20 et de ce fait déplace le produit fluide contenu dans le réservoir 20. Le volume de produit fluide déplacé correspond au volume qu'occupe le corps de pompe 11 dans le réservoir après assemblage. Le produit fluide ainsi déplacé pénètre donc dans la chambre de dosage 12 via la première ouverture 121, comme illustré par la flèche sur les figures 1 et 5. L'assemblage se faisant en position droite, la chambre de dosage 12 se remplit jusqu'au bord de la première ouverture 121, définissant ainsi la dose à distribuer. La pompe est donc amorcée dès l'assemblage, et il n'y a pas besoin de réaliser des actionnements d'amorçage avant l'utilisation du dispositif.

Lors du premier actionnement, l'utilisateur place deux doigts sur la bride radiale 153 formée sur la tête de distribution 15, et appuie avec le pouce sur le fond du réservoir 20. Lors d'un tel actionnement, le réservoir 20 est donc poussé axialement en direction de l'orifice de distribution 16, de sorte que le piston 35 coulisse dans la chambre de dosage 12. La première ouverture 121 est fermée après la petite course initiale d1 et la troisième ouverture 123 est fermée par la pression exercée par le produit fluide sur l'organe d'obturation 40 formant clapet. Le contenu de la chambre de dosage 12 est ainsi distribué à travers la seconde ouverture 122 en direction de l'orifice de distribution 16.

Si le dispositif est un bidose ou un multidose, lorsque l'utilisateur relâche la pression sur la tête de distribution 15 après le premier actionnement, le ressort 13 ramène le piston 35 vers sa position de repos. Il se crée ainsi une dépression dans la chambre de dosage 12, qui ouvre la troisième ouverture 123 pour permettre le remplissage de la chambre de dosage 12 à partir du réservoir 20 à travers cette troisième ouverture 123.

Le dispositif est alors prêt pour un second actionnement.

Ainsi, la première ouverture 121 assure le transfert de la première dose dans la chambre de dosage 12 lors de l'assemblage, et la troisième ouverture 123 assure le transfert de la seconde dose (et éventuellement d'autres doses additionnelles) dans la chambre de dosage 12 après distribution de la première dose.

Les figures 8 à 10 montrent un premier mode de réalisation avantageux, dans lequel un obturateur 39 est prévu au niveau de l'orifice de distribution 16. Cet obturateur 39 est déplaçable et/ou déformable axialement pour fermer l'orifice de distribution 16 au repos et ouvrir l'orifice de distribution 16 lors de l'actionnement. Cet obturateur 39 forme ainsi un clapet de sortie pour la pompe. Un ressort 38 peut solliciter l'obturateur 39 vers sa position d'obturation, et lors de l'actionnement, la pression du produit fluide en cours d'expulsion permet de déplacer et/ou déformer l'obturateur 39 vers sa position d'ouverture. L'obturateur 39 peut par exemple peut être formé sur l'insert 32 disposé dans la tête de distribution 15 en amont de l'orifice de distribution 16. D'autres types d'obturateurs sont aussi envisageables. En variante, on pourrait aussi prévoir un clapet de sortie dans la chambre de dosage en remplacement de l'obturateur au niveau de l'orifice de distribution.

Dans ce mode de réalisation avantageux, une membrane filtrante 60 est prévue pour filtrer l'air d'éventation qui pénètre dans la pompe après chaque actionnement.

De plus, un clip anti-actionnement amovible 100 est avantageusement disposé autour de la tête de distribution 15 pour empêcher l'actionnement pendant le transport et le stockage. Dans l'exemple représenté, le clip 100 est disposé entre le bord inférieur du corps latéral 152 et la bague de fixation 5 qui fixe le corps de pompe 11 sur le réservoir 20.

Les figures 11 à 14 montrent un deuxième mode de réalisation avantageux, comportant également un obturateur 39 et un clip anti-actionnement amovible 100 tels que décrits ci-dessus.

Dans ce mode de réalisation, il n'y a pas de membrane filtrante, mais celle-ci pourrait tout à fait également s'appliquer ici.

Le deuxième mode de réalisation, qui s'applique à un multidose, notamment un bidose, comporte des moyens de limitation de course d'actionnement, pour compenser à partir de la seconde dose le volume d'air contenu dans le canal d'expulsion 31 avant le premier actionnement. En effet, avant le premier actionnement, la chambre de dosage 12 est remplie avec du produit fluide lors de l'assemblage du distributeur 10 sur le réservoir 20, mais il reste de l'air entre la chambre de dosage 12 et l'orifice de distribution 16, notamment dans le canal d'expulsion 31. Ainsi, lors du premier actionnement, la totalité du volume de la chambre de dosage 12 n'est pas expulsé, puisqu'il va rester du produit fluide dans ces parties qui contenaient précédemment de l'air, notamment le canal d'expulsion 31. Donc, pour assurer une dose expulsée constante entre la première et la seconde dose, il est prévu de limiter la course d'actionnement du piston 35 à partir de la seconde dose. Ceci permet aussi d'isoler définitivement la première ouverture 121 de la chambre de dosage 12 après le premier actionnement.

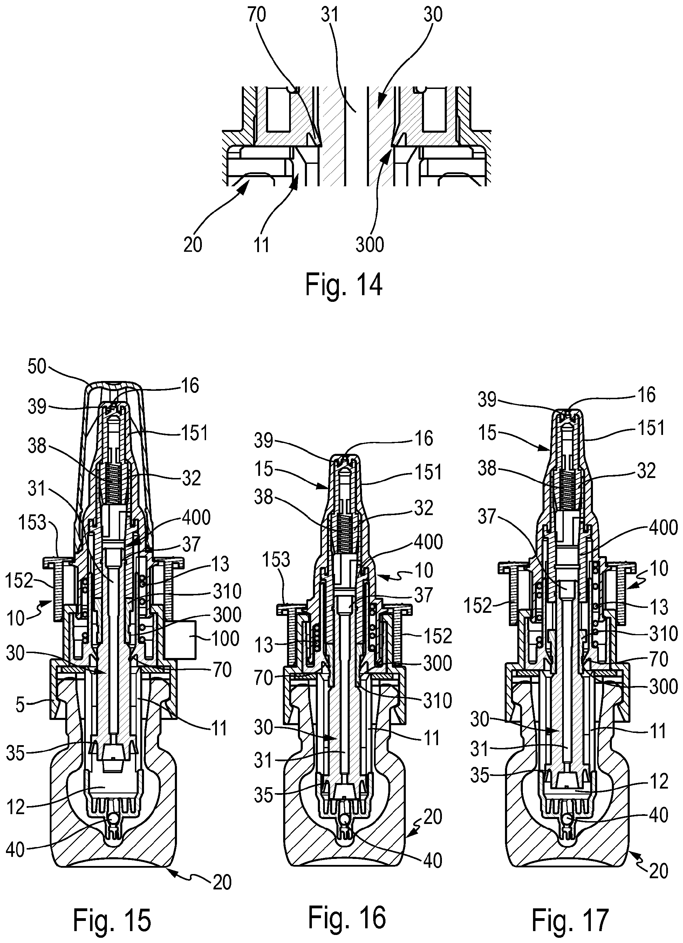

La figure 14 illustre en détail la solution du deuxième mode de réalisation, dans laquelle la tige creuse 30 qui supporte le piston 35 comporte un épaulement radial 300, qui, avant le premier actionnement, est disposé axialement au-dessus d'une lèvre radiale 70 formée sur une partie solidaire du corps de pompe 11. Lors du premier actionnement, l'épaulement 300 va passer en-dessous de ladite lèvre 70, et lorsque le piston reviendra vers sa position de repos après le premier actionnement, la lèvre 70 définira la position de butée du piston au repos, qui sera donc axialement décalée vers le bas par rapport à sa position de butée au repos avant le premier actionnement. Ainsi, à partir de la seconde dose, la course d'actionnement du piston 35 sera réduite, ce qui compensera le volume de produit fluide non distribué lors du premier actionnement en raison du volume d'air en amont de l'orifice de distribution 16.

Les figures 15 à 17 décrivent une variante avantageuse du deuxième mode de réalisation des figures 11 à 14. Dans cette variante, la course du piston 35 à partir de la seconde dose est décorrélée de la course de la tête de distribution 15, de sorte que même si le piston exerce une course d'actionnement réduite à partir de la seconde dose, la tête de distribution 15 fera toujours la même course d'actionnement que pour la première dose. Ceci est notamment avantageux pour ne pas modifier les sensations de l'utilisateur entre les première et seconde doses.

Dans cette variante, la tige creuse 30 est axialement déplaçable par rapport à la tête de distribution 15. Dans l'exemple représenté sur les figures 15 à 17, l'extrémité supérieure 37 de la tige creuse coulisse axialement dans un manchon creux 400 solidaire de la tête de distribution 15, de préférence de manière étanche.

Avantageusement, l'épaulement 300 n'est pas nécessairement formé directement sur la tige creuse 30, mais sur une bague 310 disposée autour de ladite tige creuse 30.

Ainsi, lorsque le piston revient vers sa position de repos après distribution de la première dose, comme visible sur les figures 16 et 17, la lèvre radiale 70 va coopérer avec l'épaulement 300 pour modifier la position axiale de la position de repos du piston 35. Par contre, la tête de distribution 15 sera ramenée par le ressort 13 vers sa position de repos initiale, avec l'extrémité supérieure 37 de la tige creuse coulissant axialement dans le manchon creux 400. Lorsque l'utilisateur actionnera à nouveau le dispositif pour distribuer la seconde dose, la tête de distribution 15 fera d'abord une course morte, pendant laquelle l'extrémité supérieure 37 de la tige creuse coulissera axialement dans le manchon creux 400 dans l'autre sens, puis déplacera le piston 35 sur la fin de sa course d'actionnement.

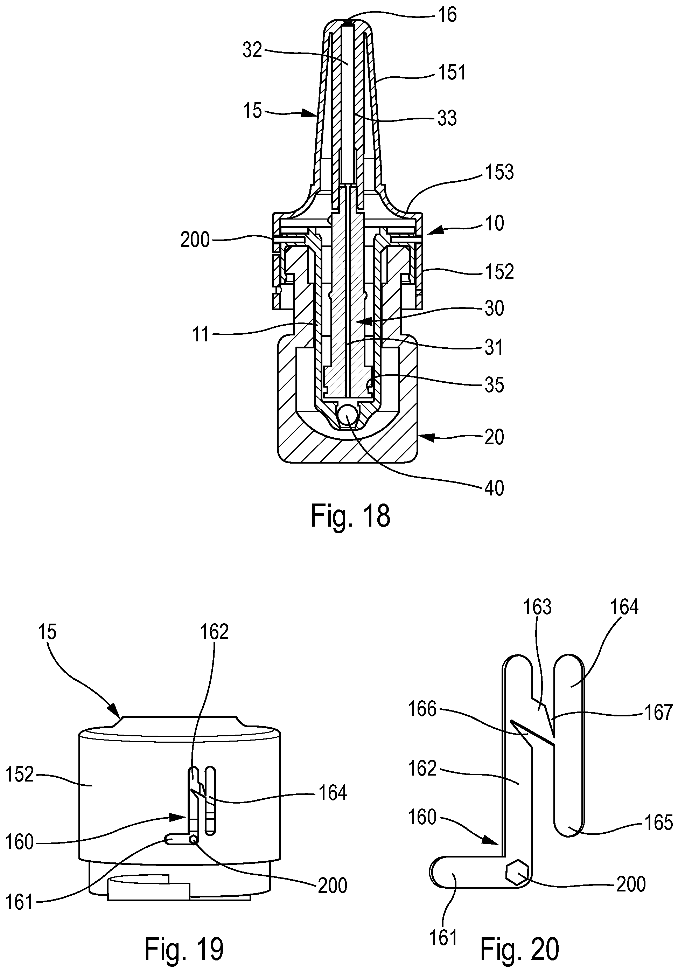

Les figures 18 à 20 illustrent un deuxième exemple.

Ce deuxième exemple ne comporte ni obturateur, ni membrane filtrante, mais ces éléments pourraient être présents aussi ici.

Ce deuxième exemple décrit un système alternatif pour d'une part réaliser l'anti-actionnement pendant le transport et le stockage, et pour d'autre part réaliser la limitation de course d'actionnement.

Comme visible en détail sur les figures 19 et 20, le corps latéral 152 de la tête de distribution 15 comporte au moins un profil de rainures 160 coopérant avec une projection radiale 200 solidaire du corps de pompe 11. Avantageusement, il peut y avoir deux profils de rainures 160 diamétralement opposés.

Le profil de rainures 160 avantageux des figures 19 et 20 comporte une rainure horizontale 161, qui se prolonge à une de ses extrémités latérales par une première rainure verticale 162. Ainsi, avant le premier actionnement, la projection radiale 200 est dans ladite rainure horizontale 161, du côté latéral opposé à la première rainure verticale 162, et par conséquent, le déplacement axial de la tête de distribution 15 par rapport au corps de pompe 11 est empêché.

Pour pouvoir réaliser le premier actionnement, l'utilisateur doit tourner la tête de distribution 15, pour faire coulisser la projection radiale 200 vers l'autre coté latéral de la rainure horizontale 161, pour se retrouver en face de la première rainure verticale 162. C'est la position représentée sur les figures 19 et 20.

Le dispositif peut alors être actionné pour distribuer la première dose.

Lors de cette première course d'actionnement, la projection radiale 200 remonte en haut de la première rainure verticale 162, en passant sur une partie de paroi déformable 166 faisant saillie dans la première rainure verticale 162.

Lorsque le piston 35 revient vers sa position de repos après le premier actionnement, la projection radiale 200 est guidée par ladite partie de paroi déformable 166 dans une rainure oblique 163 qui mène vers une seconde rainure verticale 164, parallèle à la première rainure verticale 162, mais dont l'extrémité axiale inférieure 165 est décalée axialement vers le haut par rapport à l'extrémité axiale inférieure de la première rainure verticale 162, formée par le fond de la rainure horizontale 161.

Ainsi, la position de repos du piston 35 après le premier actionnement est décalée axialement par rapport à celle avant le premier actionnement, et le second actionnement, ainsi que d'éventuels autres actionnements, auront une course d'actionnement réduite, définie par la hauteur de la seconde rainure verticale 164, inférieure à la hauteur de la première rainure verticale 162. Avantageusement, une seconde partie de paroi déformable 167 est prévue entre la rainure oblique 163 et la seconde rainure verticale 164, pour empêcher que la projection radiale 200 ne puisse revenir dans la rainure oblique 163 lors du second actionnement, ou des actionnements suivants. Typiquement, cette seconde partie de paroi déformable 167, à l'état non déformé, fait saillie dans la rainure oblique 163 et s'aligne avec la paroi latérale de la seconde rainure verticale 162.

Bien entendu, des variantes de réalisation sont envisageables, sans sortir du cadre de la présente invention, tel que défini par les revendications annexées.

## Revendications

1. Dispositif de distribution de produit fluide comprenant un distributeur (10) assemblé sur un réservoir (20) contenant du produit fluide, ledit réservoir contenant plusieurs doses de produit fluide distribuées en plusieurs actionnements successifs, ledit distributeur (10) comportant un corps de pompe (11) contenant une chambre de dosage (12), et une tête de distribution (15) comportant un orifice de distribution (16), ledit orifice de distribution (16) comportant un obturateur (39) déplaçable et/ou déformable entre une position de fermeture et une position d'ouverture, ledit obturateur (39) étant élastiquement sollicité vers sa position de fermeture et étant adapté à s'ouvrir lors de l'actionnement, ladite tête de distribution (15) étant déplaçable axialement par rapport audit corps de pompe (11) entre une position de repos et une position d'actionnement, un piston (35), solidaire de ladite tête de distribution (15), étant adapté à coulisser dans ladite chambre de dosage (12) entre une position de repos et une position d'actionnement, **caractérisé en ce que** ladite chambre de dosage (12) comporte trois ouvertures (121, 122, 123), une première ouverture (121) reliant ladite chambre de dosage (12) audit réservoir (20) lorsque ledit piston (35) est en position de repos avant le premier actionnement, une seconde ouverture (122) reliant ladite chambre de dosage (12) audit orifice de distribution (16) et une troisième ouverture (123) prévue à l'extrémité axiale inférieure dudit corps de pompe (11), lesdites première et troisième ouvertures (121, 123) étant isolées de ladite chambre de dosage (12) lorsque ledit piston (35) se déplace hors de sa position de repos, ladite chambre de dosage (12) étant remplie à travers ladite première ouverture (121) lorsque ledit distributeur (10) est assemblé sur ledit réservoir (20).

2. Dispositif selon la revendication 1, dans lequel ladite première ouverture (121) est réalisée dans une partie cylindrique dudit corps de pompe (11) dans laquelle ledit piston (35) coulisse lors de l'actionnement.

3. Dispositif selon la revendication 1 ou 2, dans lequel un bord inférieur de ladite première ouverture (121) est disposé au repos à une distance (d1) dudit piston (35), de sorte que, lors de l'actionnement, ladite première ouverture (121) est fermée après une course initiale correspondant à ladite distance (d1).

4. Dispositif selon la revendication 3, dans lequel ladite distance (d1) forme moins de 30% de la course d'actionnement totale dudit piston (35), de préférence environ 20%.

5. Dispositif selon la revendication 2, dans lequel ladite première ouverture (121) comporte une ou plusieurs fentes formée(s) dans la partie cylindrique dudit corps de pompe (11).

6. Dispositif selon l'une quelconque des revendications précédentes, dans lequel une tige creuse (30) est disposée dans ladite tête de distribution (15), ladite tige creuse (30) définissant un canal d'expulsion (31) reliant lors de l'actionnement ladite chambre de dosage (12) audit orifice de distribution (16), ladite tige creuse (30) comportant ledit piston (35).

7. Dispositif selon la revendication 6, dans lequel ledit piston (35) est formé par un joint (350), notamment un joint torique, assemblé sur ladite tige creuse (30).

8. Dispositif selon l'une quelconque des revendications précédentes, dans lequel ladite troisième ouverture (123) comporte un organe d'obturation (40), tel qu'une bille, formant un clapet d'entrée pour ladite chambre de dosage (12) permettant le remplissage de celle-ci après chaque actionnement,

9. Dispositif selon l'une quelconque des revendications précédentes, comportant une membrane filtrante (60) pour filtrer l'air d'éventation après chaque actionnement.

10. Dispositif selon l'une quelconque des revendications précédentes, comportant des moyens anti-actionnement (100 ; 200, 161) pour empêcher le déplacement axial de ladite tête de distribution (15) par rapport audit corps de pompe (11).

11. Dispositif selon la revendication 10, dans lequel lesdits moyens anti-actionnement comportent un clip amovible (100) disposé autour dudit distributeur (10) avant le premier actionnement.

12. Dispositif selon la revendication 10, dans lequel lesdits moyens anti-actionnement comportent une projection radiale (200) solidaire dudit corps de pompe (11) coopérant avec une rainure horizontale (161) d'un profil de rainures (160) solidaire de ladite tête de distribution (15).

13. Dispositif selon l'une quelconque des revendications précédentes, comportant des moyens de limitation de course d'actionnement (70, 300 ; 160, 200) pour réduire la course d'actionnement dudit piston (35) après la distribution de la première dose en décalant axialement la position de repos dudit piston (35) après le premier actionnement.

14. Dispositif selon la revendication 13, dans lequel lesdits moyens de limitation de course d'actionnement comportent une lèvre radiale (70) solidaire dudit corps de pompe (11) coopérant avec un épaulement radial (300) solidaire dudit piston (35).

15. Dispositif selon la revendication 13, dans lequel lesdits moyens de limitation de course d'actionnement comportent une projection radiale (200) solidaire dudit corps de pompe (11) coopérant avec un profil de rainures (160) solidaire de ladite tête de distribution (15).

16. Dispositif selon la revendication 15, dans lequel ledit profil de rainures (160) comporte une première rainure verticale (162) et une seconde rainure verticale (164) parallèle, reliées par une rainure oblique (163), la position de repos dudit piston (35) avant le premier actionnement correspondant à ladite projection radiale (200) disposée dans l'extrémité axiale inférieure de ladite première rainure verticale (162) et la position de repos dudit piston (35) après le premier actionnement correspondant à ladite projection radiale (200) disposée dans l'extrémité axiale inférieure (165) de ladite seconde rainure verticale (164), lesdites extrémités axiales inférieures (161, 165) étant décalées axialement l'une de l'autre.

17. Dispositif selon la revendication 16, dans lequel ladite première rainure verticale (162) comporte une partie de paroi déformable (166) adaptée à guider ladite projection radiale (200) vers ladite rainure oblique (163) lorsque ledit piston (35) revient vers sa position de repos après distribution de la première dose.

18. Dispositif selon la revendication 15 ou 16, dans lequel ladite rainure oblique (163) comporte une seconde partie de paroi déformable (167) adaptée à empêcher ladite projection radiale (200) de revenir dans ladite rainure oblique (163) lorsque ladite projection radiale (200) est entrée dans ladite seconde rainure verticale (164).

19. Dispositif selon l'une quelconque des revendications précédentes, dans lequel ledit réservoir contient deux doses de produit fluide distribuées en deux actionnements successifs.

20. Procédé d'amorçage d'un dispositif de distribution de produit fluide, comportant les étapes suivantes:
- fournir un réservoir (20) contenant au moins deux doses de produit fluide,
- fournir un distributeur (10) comportant un corps de pompe (11) contenant une chambre de dosage (12), et une tête de distribution (15) comportant un orifice de distribution (16), ledit orifice de distribution (16) comportant un obturateur (39) déplaçable et/ou déformable entre une position de fermeture et une position d'ouverture, ledit obturateur (39) étant élastiquement sollicité vers sa position de fermeture et étant adapté à s'ouvrir lors de l'actionnement, ladite tête de distribution (15) étant déplaçable axialement par rapport audit corps de pompe (11) entre une position de repos et une position d'actionnement, un piston (35), solidaire de ladite tête de distribution (15), étant adapté à coulisser dans ladite chambre de dosage (12) entre une position de repos et une position d'actionnement,
**caractérisé en ce que** ladite chambre de dosage (12) comporte trois ouvertures (121, 122, 123), une première ouverture (121) reliant ladite chambre de dosage (12) audit réservoir (20) lorsque ledit piston (35) est en position de repos avant le premier actionnement, une seconde ouverture (122) reliant ladite chambre de dosage (12) audit orifice de distribution (16) et une troisième ouverture (123) prévue à l'extrémité axiale inférieure dudit corps de pompe (11), lesdites première et troisième ouvertures (121, 123) étant isolées de ladite chambre de dosage (12) lorsque ledit piston (35) se déplace hors de sa position de repos,
et **en ce que** ledit procédé comporte l'étape d'assembler ledit distributeur (10) sur ledit réservoir (20), en insérant ledit corps de pompe (11) dans ledit réservoir (20) avec ledit piston (35) en position de repos, cette insertion déplaçant le produit fluide contenu dans ledit réservoir (20), ledit produit fluide déplacé pénétrant dans ladite chambre de dosage (12) à travers ladite première ouverture (121), pour ainsi amorcer le dispositif.

## Patentansprüche

1. Vorrichtung zur Abgabe eines flüssigen Produkts, aufweisend einen auf einem Behälter (20) montierten Spender (10), der das flüssige Produkt enthält, wobei der Behälter mehrere Dosen des flüssigen Produkts enthält, die in mehreren aufeinanderfolgenden Betätigungen abgegeben werden, wobei der Spender (10) einen Pumpenkörper (11), der eine Dosierkammer (12) enthält, und einen Abgabekopf (15) aufweist, der eine Abgabeöffnung (16) aufweist, wobei die Abgabeöffnung (16) einen Verschlusskörper (39) aufweist, der zwischen einer Schließstellung und einer Offenstellung verschiebbar und/oder verformbar ist, wobei der Verschlusskörper (39) elastisch in Richtung seiner Schließstellung vorgespannt ist und dazu eingerichtet ist, sich bei der Betätigung zu öffnen, wobei der Abgabekopf (15) axial relativ zu dem Pumpenkörper (11) zwischen einer Ruhestellung und einer Betätigungsstellung beweglich ist, wobei ein mit dem Abgabekopf (15) verbundener Kolben (35) dazu eingerichtet ist, in der Dosierkammer (12) zwischen einer Ruhestellung und einer Betätigungsstellung zu gleiten,
**dadurch gekennzeichnet, dass**
die Dosierkammer (12) drei Öffnungen (121, 122, 123) aufweist, eine erste Öffnung (121), die die Dosierkammer (12) mit dem Behälter (20) verbindet, wenn sich der Kolben (35) in der Ruhestellung vor der ersten Betätigung befindet, eine zweite Öffnung (122), die die Dosierkammer (12) mit der Abgabeöffnung (16) verbindet, und eine dritte Öffnung (123), die an dem axial unteren Ende des Pumpenkörpers (11) vorgesehen ist, wobei die erste und die dritte Öffnung (121, 123) von der Dosierkammer (12) isoliert sind, wenn sich der Kolben (35) aus seiner Ruhestellung heraus bewegt, wobei die Dosierkammer (12) durch die erste Öffnung (121) gefüllt wird, wenn der Spender (10) auf den Behälter (20) montiert ist.

2. Vorrichtung nach Anspruch 1, wobei die erste Öffnung (121) in einem zylindrischen Teil des Pumpenkörpers (11) ausgebildet ist, in dem der Kolben (35) bei Betätigung gleitet.

3. Vorrichtung nach Anspruch 1 oder 2, wobei ein unterer Rand der ersten Öffnung (121) im Ruhezustand in einem Abstand (d1) von dem Kolben (35) angeordnet ist, sodass die erste Öffnung (121) während der Betätigung nach einem Anfangshub, der dem Abstand (d1) entspricht, geschlossen wird.

4. Vorrichtung nach Anspruch 3, wobei der Abstand (d1) weniger als 30 % des gesamten Betätigungshubs des Kolbens (35) beträgt, vorzugsweise etwa 20 %.

5. Vorrichtung nach Anspruch 2, wobei die erste Öffnung (121) einen oder mehrere in dem zylindrischen Teil des Pumpenkörpers (11) ausgebildete Schlitze aufweist.

6. Vorrichtung nach einem der vorhergehenden Ansprüche, wobei ein Hohlstab (30) in dem Abgabekopf (15) angeordnet ist, wobei der Hohlstab (30) einen Ausstoßkanal (31) definiert, der bei der Betätigung die Dosierkammer (12) mit der Abgabeöffnung (16) verbindet, wobei der Hohlstab (30) den Kolben (35) aufweist.

7. Vorrichtung nach Anspruch 6, wobei der Kolben (35) durch eine Dichtung (350), insbesondere einen O-Ring, gebildet ist, die auf dem Hohlstab (30) angebracht ist.

8. Vorrichtung nach einem der vorhergehenden Ansprüche, wobei die dritte Öffnung (123) ein Verschlusselement (40), wie eine Kugel, aufweist, das ein Einlassventil für die Dosierkammer (12) bildet, um deren Befüllung nach jeder Betätigung zu ermöglichen.

9. Vorrichtung nach einem der vorhergehenden Ansprüche, die eine Filtermembran (60) zum Filtern der Entlüftungsluft nach jeder Betätigung aufweist.

10. Vorrichtung nach einem der vorhergehenden Ansprüche, die Betätigungssperrmittel (100; 200, 161) aufweist, um die axiale Bewegung des Abgabekopfs (15) relativ zu dem Pumpenkörper (11) zu verhindern.

11. Vorrichtung nach Anspruch 10, wobei die Betätigungssperrmittel einen abnehmbaren Clip (100) aufweisen, der vor der ersten Betätigung um den Spender (10) herum angeordnet ist.

12. Vorrichtung nach Anspruch 10, wobei die Betätigungssperrmittel einen radialen Vorsprung (200) aufweisen, der mit dem Pumpenkörper (11) verbunden ist und mit einer horizontalen Nut (161) eines Nutprofils (160) zusammenwirkt, das mit dem Abgabekopf (15) verbunden ist.

13. Vorrichtung nach einem der vorhergehenden Ansprüche, die Hubbegrenzungsmittel (70, 300; 160, 200) aufweist, um den Betätigungshub des Kolbens (35) nach der Abgabe der ersten Dosis zu verringern, indem die Ruhestellung des Kolbens (35) nach der ersten Betätigung axial versetzt wird.

14. Vorrichtung nach Anspruch 13, wobei die Hubbegrenzungsmittel eine radiale Lippe (70) aufweisen, die mit dem Pumpenkörper (11) verbunden ist und mit einem radialen Absatz (300) zusammenwirkt, der mit dem Kolben (35) verbunden ist.

15. Vorrichtung nach Anspruch 13, wobei die Hubbegrenzungsmittel einen radialen Vorsprung (200) aufweisen, der mit dem Pumpenkörper (11) verbunden ist und mit einem Nutprofil (160) zusammenwirkt, das mit dem Abgabekopf (15) verbunden ist.

16. Vorrichtung nach Anspruch 15, wobei das Nutprofil (160) eine erste vertikale Nut (162) und eine zweite parallele vertikale Nut (164) aufweist, die durch eine schräge Nut (163) miteinander verbunden sind, wobei die Ruhestellung des Kolbens (35) vor der ersten Betätigung dem radialen Vorsprung (200) entspricht, der sich in dem axial unteren Ende der ersten vertikalen Nut (162) befindet, und die Ruhestellung des Kolbens (35) nach der ersten Betätigung dem radialen Vorsprung (200) entspricht, der sich in dem axial unteren Ende (165) der zweiten vertikalen Nut (164) befindet, wobei die axial unteren Enden (161, 165) axial gegeneinander versetzt sind.

17. Vorrichtung nach Anspruch 16, wobei die erste vertikale Nut (162) einen verformbaren Wandabschnitt (166) aufweist, der dazu eingerichtet ist, den radialen Vorsprung (200) zur schrägen Nut (163) hin zu führen, wenn der Kolben (35) nach der Abgabe der ersten Dosis in seine Ruhestellung zurückkehrt.

18. Vorrichtung nach Anspruch 15 oder 16, wobei die schräge Nut (163) einen zweiten verformbaren Wandabschnitt (167) aufweist, der dazu eingerichtet ist, zu verhindern, dass der radiale Vorsprung (200) in die schräge Nut (163) zurückkehrt, wenn der radiale Vorsprung (200) in die zweite vertikale Nut (164) eingetreten ist.

19. Vorrichtung nach einem der vorhergehenden Ansprüche, wobei der Behälter zwei Dosen des flüssigen Produkts enthält, die in zwei aufeinanderfolgenden Betätigungen abgegeben werden.

20. Verfahren zum in Betrieb nehmen einer Vorrichtung zur Abgabe eines flüssigen Produkts mit folgenden Schritten:
- Bereitstellen eines Behälters (20), der mindestens zwei Dosen des flüssigen Produkts enthält,
- Bereitstellen eines Spenders (10), der einen Pumpenkörper (11), der eine Dosierkammer (12) enthält, und einen Abgabekopf (15) aufweist, der eine Abgabeöffnung (16) aufweist, wobei die Abgabeöffnung (16) einen Verschlusskörper (39) aufweist, der zwischen einer Schließstellung und einer Offenstellung verschiebbar und/oder verformbar ist, wobei der Verschlusskörper (39) elastisch in Richtung seiner Schließstellung vorgespannt und dazu eingerichtet ist, sich bei der Betätigung zu öffnen, wobei der Abgabekopf (15) axial relativ zu dem Pumpenkörper (11) zwischen einer Ruhestellung und einer Betätigungsstellung beweglich ist, wobei ein mit dem Abgabekopf (15) verbundener Kolben (35) dazu eingerichtet ist, in der Dosierkammer (12) zwischen einer Ruhestellung und einer Betätigungsstellung zu gleiten,
**dadurch gekennzeichnet, dass**
die Dosierkammer (12) drei Öffnungen (121, 122, 123) aufweist, eine erste Öffnung (121), die die Dosierkammer (12) mit dem Behälter (20) verbindet, wenn sich der Kolben (35) in der Ruhestellung vor der ersten Betätigung befindet, eine zweite Öffnung (122), die die Dosierkammer (12) mit der Abgabeöffnung (16) verbindet, und eine dritte Öffnung (123), die an dem axial unteren Ende des Pumpenkörpers (11) vorgesehen ist, wobei die erste und die dritte Öffnung (121, 123) von der Dosierkammer (12) isoliert sind, wenn sich der Kolben (35) aus seiner Ruhestellung heraus bewegt, und dass das Verfahren den Schritt umfasst, den Spender (10) auf den Behälter (20) zu montieren, indem der Pumpenkörper (11) in den Behälter (20) mit dem Kolben (35) in der Ruhestellung eingeführt wird, wobei durch dieses Einführen das im Behälter (20) enthaltene flüssige Produkt verdrängt wird, und das verdrängte flüssige Produkt durch die erste Öffnung (121) in die Dosierkammer (12) eindringt, um so die Vorrichtung in Betrieb zu nehmen.

## Claims

1. Device for dispensing a fluid product comprising a dispenser (10) assembled on a reservoir (20) containing fluid product, said reservoir containing several doses of fluid product dispensed in several successive actuations, said dispenser (10) comprising a pump body (11) containing a dosing chamber (12), and a dispensing head (15) comprising a dispensing aperture (16), said dispensing aperture (16) comprising a stopper (39) that is movable and/or deformable between a closed position and an open position, said stopper (39) being resiliently biased towards its closed position and being adapted to open during actuation, said dispensing head (15) being axially movable with respect to said pump body (11) between a rest position and an actuation position, a piston (35), secured to said dispensing head (15), being adapted to slide in said dosing chamber (12) between a rest position and an actuation position, **characterised in that** said dosing chamber (12) comprises three openings (121, 122, 123), a first opening (121) connecting said dosing chamber (12) to said reservoir (10) when said piston (35) is in the rest position prior to the first actuation, a second opening (122) connecting said dosing chamber (12) to said dispensing aperture (16) and a third opening (123) provided at the lower axial end of said pump body (11), said first and third openings (121, 123) being isolated from said dosing chamber (12) when said piston (345) moves out of its rest position, said dosing chamber (12) being filled through said first opening (121) when said dispenser (10) is assembled on said reservoir (20).

2. Device according to claim 1, wherein said first opening (121) is made in the cylindrical portion of said pump body (11) in which the piston (35) slides during actuation.

3. Device according to claim 1 or 2, wherein a lower edge of said first opening (121) is disposed at rest at a distance (d1) from said piston (35), such that during actuation, said first opening (121) is closed after an initial stroke corresponding to said distance (d1).

4. Device according to claim 3, wherein said distance (d1) forms less than 30% of the total actuation stroke of said piston (35), preferably around 20%.

5. Device according to claim 2, wherein said first opening (121) comprises one or more slots formed in the cylindrical portion of said pump body (11).

6. Device according to any one of preceding claims, wherein a hollow rod (30) is disposed in said dispensing head (15), said hollow rod (30) defining an expulsion channel (31) connecting, during actuation, said dosing chamber (12) to said dispensing aperture (16), said hollow rod (30) comprising said plunger (35).

7. Device according to claim 6, wherein said piston (35) is formed by a seal (350), in particular an O-ring, assembled to said hollow rod (30).

8. Device according to any one of the preceding claims, wherein said third opening (123) comprises a stopper member (40), such as a ball, forming an inlet valve for said dosing chamber (12) for filling it after each actuation,

9. Device according to any one of the preceding claims, comprising a filtering membrane (60) for filtering the vent air after each actuation.

10. Device according to any one of the preceding claims, comprising anti-actuation means (100; 200, 161) for preventing the axial movement of said dispensing head (15) relative to said pump body (11).

11. Device according to claim 10, wherein said anti-actuation means comprise a removable clip (100) arranged around said dispenser (10) before the first actuation.

12. Device according to claim 10, wherein said anti-actuation means comprise a radial projection (200) secured to said pump body (11) co-operating with a horizontal groove (161) of a groove profile (160) secured to said dispensing head (15).

13. Device according the any one of the preceding claims, comprising actuation stroke limitation means (70, 300; 160, 200) for reducing the actuation stroke of said piston (35) after the first dose has been dispensed by axially shifting the rest position of said piston (35) after the first actuation.

14. Device according to claim 13, wherein said actuation stroke limitation means comprise a radial lip secured to said pump body (11) co-operating with a radial shoulder (300) secured to said piston (35).

15. Device according to claim 13, wherein said actuation stroke actuation means comprise a radial projection (200) secured to said pump body (11) co-operating with a groove profile (160) secured to said dispensing head (15).

16. Device according to claim 15, wherein said groove profile (160) comprises a first vertical groove (162) and a second parallel vertical groove (164), connected by an oblique groove (163), the rest position of said piston (35) before the first actuation corresponding to said radial projection (200) disposed in the lower axial end of said first vertical groove (162) and the rest position of said piston (35) after the first actuation corresponding to said radial projection (200) disposed in the lower axial end (165) of said second vertical groove (164), said lower axial ends (161, 165) being axially offset from one another.

17. Device according to claim 16, wherein said first vertical groove (162) comprises a deformable wall portion (166) adapted to guide said radial projection (200) towards said oblique groove (163) when said piston (35) returns towards its rest position after the first dose has been dispensed.

18. Device according to claim 15 or claim 16, wherein said oblique groove (163) comprises a second deformable wall portion (167) suitable for preventing said radial projection (200) from returning into said oblique groove (163) when said radial projection (200) has entered said second vertical groove (164).

19. Device according to any one of claims preceding, wherein said vessel contains two doses of fluid product dispensed in two successive actuations.

20. Method for priming a device for dispensing a fluid product, comprising the following steps:
- providing a reservoir (20) which contains at least two doses of fluid product;
- providing a dispenser (10) comprising a pump body (11) containing a dosing chamber (12), and a dispensing head (15) comprising a dispensing aperture (16), said dispensing aperture (16) comprising a stopper (39) that is movable and/or deformable between a closed position and an open position, said stopper (39) being resiliently biased towards its closed position and being adapted to open during actuation, said dispensing head (15) being axially movable with respect to said pump body (11) between a rest position and an actuation position, a piston (35), secured to said dispensing head (15), being adapted to slide in said dosing chamber (12) between a rest position and an actuation position, **characterised in that** said dosing chamber (12) comprises three openings (121, 122, 123), a first opening (121) connecting said dosing chamber (12) to said reservoir (20) when said piston (35) is in the rest position prior to the first actuation, a second opening (122) connecting said dosing chamber (12) to said dispensing aperture (16) and a third opening (123) provided at the lower axial end of said pump body (11), said first and third openings (121, 123) being isolated from said dosing chamber (12) when said piston (35) moves out of its rest position, and **in that** said method comprises the step of joining said dispenser (10) to said reservoir (20), by inserting said pump body (11) in said reservoir (20) with said plunger (35) in the rest position, this insertion moving the fluid product contained in said reservoir (20), said moved fluid product penetrating inside said dosing chamber (12) through said first opening (121), to thus prime the device.
